# EUROPEAN PATENT SPECIFICATION

(11) **EP 1 872 812 B1**
(45) Date of publication and mention of the grant of the patent: **18.11.2015**
(21) Application number: 07111217.1
(22) Date of filing: 27.06.2007
(51) Int. Cl.: A61M 1/14, A61B 5/1486, A61M 1/16

(54) **Dialysis machine with glycaemia control**
Dialysemaschine mit Glykämiekontrolle
Machine de dialyse avec contrôle de la glycémie

(30) Priority: 27.06.2006 IT BO20060493
(43) Date of publication of application: 02.01.2008
(73) Proprietor: Bellco S.r.l., Mirandola (IT)
(72) Inventor: Cianciavicchia, Domenico, 64040, Cavuccio (IT); Fiorenzi, Andrea, 41013, Castelfranco Emilia (IT); Baroni, Paolo, 46035, Ostiglia (IT); Cavani, Silvia, 41100, Modena (IT)
(74) Representative: Bergadano, Mirko

(56) References cited:
- WO-A-98/19592
- WO-A-2004/089440
- WO-A-2005/028001
- WO-A1-2004/024300
- US-A1- 2006 052 745

## Description

The present invention relates to a dialysis machine with glycaemia control.

Dialysis is a blood-purifying method for restoring the water-salt balance of the blood, and for eliminating surplus water and toxins accumulating in the body as a result of renal failure, by releasing them to an electrolytic liquid similar to that of normal plasma not containing them, and which hereinafter is referred to as a "dialysis solution". The method itself comprises feeding blood from the patient's arm along a so-called artery line into the dialyzer, and feeding the purified blood from the dialyzer along a so-called vein line back to the patient.

In hemodiafiltration, to which the following description refers purely by way of example, blood is purified by both diffusion and convection. Purification by diffusion is achieved by balancing the concentrations of the blood and the dialysis solution flowing from opposite sides of a semipermeable membrane; while purification by convection is achieved by forming a pressure gradient between the dialysis solution compartment and the blood compartment, in favour of the latter, so that plasma flows through the semipermeable membrane, taking with it also any toxins dissolved in it.

The dialysis solution contains none of the substances to be eliminated from the blood, such as urea, uric acid, creatinine, phosphorous, etc., but contains a precise quantity of other substances to be balanced, such as sodium, calcium, magnesium, potassium, etc.

As is known, many patients requiring dialysis treatment suffer from diabetes, in which case, it is vital to also ensure a correct balance of the glucose in the blood during dialysis treatment.

WO2004/089440 discloses a blood purification device comprising a blood purification element that is divided into two chambers by a semi-permeable membrane. The device further comprises a sensor fitted to a circuit portion in which the spent dialysis solution comprising components of the dialysis-treated blood circulates. The device permits the determination of the concentration of the second substance in the blood during haemodialysis by measurements carried out on the dialysate, without the need for intervention in the course of dialysis. US2006/052745 discloses a method and an apparatus for detecting an analyte in blood. In particular, the apparatus comprises a biosensor suitable for direct contact with blood and capable of measuring glucose and/or other analytes in a variety of applications. In addition, the apparatus comprises a venous flow device having a lumen within the sensor is suspended. WO2004/024300 discloses a compact ultrafiltration device and a method for generating an ultrafiltrate, both of which can be used for a variety of applications, including filtering blood, diagnostic applications, and as a bioreactor.

It is an object of the present invention to provide a dialysis machine designed to control the glucose level in the patient's blood during dialysis treatment.

According to the present invention, there is provided a dialysis machine as claimed in Claim 1 or in Claim 2.

In a first preferred embodiment of the dialysis machine according to the present invention, the measuring device is fitted to the dialysis solution outlet branch.

In a second preferred embodiment, the dialysis machine according to the present invention comprises an isolated-ultrafiltration device located along the artery line of the patient's blood, and from which originates a branch line carrying the blood components and fitted with the glucose concentration measuring device.

Non-limiting embodiments of the invention will be described by way of example with reference to the accompanying drawings, in which:
Figure 1 shows a schematic of part of a first preferred embodiment of the dialysis machine according to the present invention;
Figure 2 shows a schematic of part of a second preferred embodiment of the dialysis machine according to the present invention.

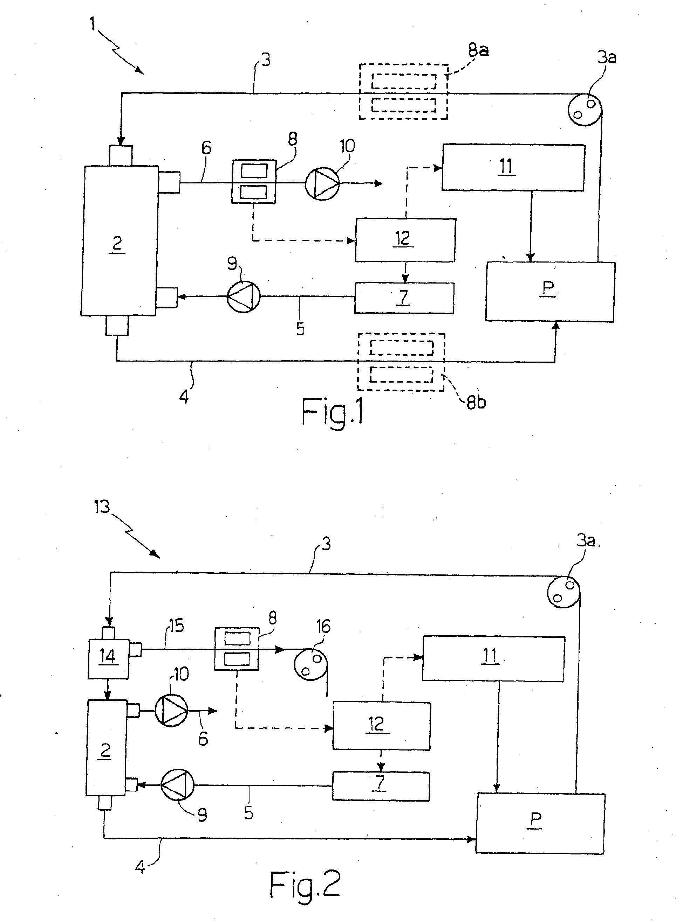

Number 1 in Figure 1 indicates as a whole a first preferred embodiment of the dialysis machine (only shown partly) according to the present invention.

Machine 1 comprises a haemodialysis filter 2 (known and not described in detail); an artery line 3 carrying blood from a patient P to filter 2; a pump 3a fitted to artery line 3 to ensure blood flow; a vein line 4 carrying blood from filter 2 to patient P; an inlet branch 5 and outlet branch 6 carrying a dialysis solution to and from filter 2 respectively; a dialysis solution preparation device 7 connected to inlet branch 5; a glucose concentration measuring device 8 fitted to outlet branch 6; two pumps 9 and 10 fitted to dialysis solution inlet branch 5 and outlet branch 6 respectively; an infusion device 11 for injecting patient P with insulin; and a central control unit 12.

Central control unit 12 is connected to, and supplied with data from, glucose concentration measuring device 8, is connected to dialysis solution preparation device 7 to meter glucose into the solution as a function of the data from measuring device 8, and is connected to infusion device 11 to inject patient P with a quantity of insulin as a function of the data from measuring device 8.

Figure 1 shows two possible variations, in which the glucose concentration measuring device is indicated by dash lines. More specifically, a first variation comprises a measuring device 8a fitted along artery line 3, and a second possible variation comprises a measuring device 8b fitted along vein line 4.

Number 13 in Figure 2 indicates as a whole a second preferred embodiment of the dialysis machine (only shown partly) according to the present invention. Parts of machine 13 identical to those of machine 1 are indicated using the same reference numbers, with no further description.

Machine 13 substantially differs from machine 1 by comprising an isolated-ultrafiltration device 14 located along artery line 3 to produce a quantity of ultrafiltrated plasma, which is fed into an artery branch line 15 fitted with a pump 16. Artery branch line 15 normally comprises an adsorption purifying device, and reconnects with artery line 3 along the portion extending between isolated-ultrafiltration device 14 and haemodialysis filter 2.

Alternatively, plasma water as opposed to ultrafiltrated plasma may be fed into artery branch line 15.

As shown in Figure 2, in machine 13, glucose concentration measuring device 8 is fitted to artery branch line 15 and connected, as in machine 1, to central control unit 12.

Measuring device 8 has a measuring sensitivity of 20-600 mg/dL, and operates on the basis of an electrochemical principle using the transformation of glucose by an oxidase glucose enzyme and the consequent production of free electrons. As will be obvious from the above description, the type of measuring device is in no way binding and therefore non-limiting.

The dialysis machine according to the present invention provides for continuously monitoring the glucose concentration in the dialysis patient's blood. Moreover, machine 1 intervenes immediately to avoid subjecting the dialysis patient to clinical complications caused by too high or too low glucose levels in the blood. For which purpose, the machine comprises an intervention system comprising infusion device 11 and dialysis solution preparation device 7.

The machine according to the present invention may comprise an intervention system comprising infusion device 11 only, or preparation device 7 only.

In actual use, during dialysis treatment, central control unit 12 receives the glucose values in the patient's blood from measuring device 8, and, after comparing them with preset values, accordingly controls preparation device 7 and/or infusion device 11 on the basis of appropriate algorithms.

## Claims

1. A dialysis machine (1; 13) comprising a haemodialysis filter (2); an inlet branch (5) carrying a dialysis solution to said filter (2); an outlet branch (6) carrying the dialysis solution from said filter (2); an artery line (3) carrying blood from a patient (P) to the filter (2); a vein line (4) carrying blood from the filter (2) to the patient (P); a number of pumps (3a, 9, 10, 16) for circulating both the blood and the dialysis solution; an isolated-ultrafiltration device (14) located along the artery line (3) of the patient's blood, and from which originates a branch line (15) wherein ultrafiltrated plasma or plasma water circulates; and a glucose concentration measuring device (8) fitted to said branch line (15); said machine being **characterized by** comprising a central control unit (12) connected to both said measuring device (8) to receive data relative to the glucose concentration in the blood, and to an intervention system (7, 11) for restoring the correct glucose concentration in the patient's blood as a function of the data from the measuring device (8); said intervention system comprising a dialysis solution preparation device (7) for metering glucose into the dialysis solution and an insulin infusion device (11) for injecting insulin into the patient (P) undergoing dialysis.

2. A dialysis machine (1; 13) comprising a haemodialysis filter (2); an inlet branch (5) carrying a dialysis solution to said filter (2); an outlet branch (6) carrying the dialysis solution from said filter (2); an artery line (3) carrying blood from a patient (P) to the filter (2); a vein line (4) carrying blood from the filter (2) to the patient (P); a number of pumps (3a, 9, 10, 16) for circulating both the blood and the dialysis solution; and a glucose concentration measuring device (8; 8a; 8b) fitted to said vein line (3) or to said arterial line (4) or to dialysate outflow line; said machine being **characterized by** comprising a central control unit (12) connected to both said measuring device (8) to receive data relative to the glucose concentration in the blood, and to an intervention system (7, 11) for restoring the correct glucose concentration in the patient's blood as a function of the data from the measuring device (8); said intervention system comprising a dialysis solution preparation device (7) for metering glucose into the dialysis solution and an insulin infusion device (11) for injecting insulin into the patient (P) undergoing dialysis.

## Patentansprüche

1. Dialysegerät (1; 13), umfassend einen Hämodialysefilter (2); einen Einlasszweig (5), der eine Dialyselösung zu dem Filter (2) fördert; einen Auslasszweig (6), der eine Dialyselösung von dem Filter (2) weg fördert; eine Arterienleitung (3), die Blut von einem Patienten (P) zu dem Filter (2) fördert; eine Venenleitung (4), die Blut von dem Filter (2) zu dem Patienten (P) fördert; eine Anzahl an Pumpen (3a, 9, 10, 16) zur Zirkulation sowohl des Blutes als auch der Dialyselösung; eine getrennte Ultrafiltrationsvorrichtung (14), die entlang der Arterienleitung (3) des Blutes des Patienten angeordnet ist und von welcher eine Abzweigung (15) hervorgeht, worin ultrafiltriertes Plasma oder Plasmawasser zirkuliert; und eine Glucosekonzentrations-Messvorrichtung (8), die an die Abzweigung (15) angebaut ist; wobei das Gerät **dadurch gekennzeichnet ist, dass** es eine zentrale Steuereinheit (12) umfasst, die sowohl mit der Messvorrichtung (8) verbunden ist, um Daten bezüglich der Glucosekonzentration im Blut zu erhalten, als auch mit einem Eingriffsystem (7,11) zur Wiederherstellung der korrekten Glucosekonzentration im Blut des Patienten als Funktion der Daten von der Messvorrichtung (8); wobei das Eingriffsystem eine Dialyselösungs-Vorbereitungsvorrichtung (7) zur Dosierung der Glucose in die Dialyselösung und eine Insulininfusionsvorrichtung (11) zur Insulininjektion in den Patienten (P), der die Dialysebehandlung erhält, umfasst.

2. Dialysegerät (1; 13), umfassend einen Hämodialysefilter (2); einen Einlasszweig (5), der eine Dialyselösung zu dem Filter (2) fördert; einen Auslasszweig (6), der die Dialyselösung von dem Filter (2) weg fördert; eine Arterienleitung (3), die Blut von einem Patienten (P) zu dem Filter (2) fördert; eine Venenleitung (4), die Blut von dem Filter (2) zu dem Patienten (P) fördert; eine Anzahl an Pumpen (3a, 9, 10, 16) zur Zirkulation sowohl des Blutes als auch der Dialyselösung; und eine Glucosekonzentrations-Messvorrichtung (8; 8a; 8b), die an die Venenleitung (4) oder an die Arterienleitung (3) oder an die Dialysat-Abflussleitung angebaut ist; wobei das Gerät dadurch charakterisiert ist, dass es eine zentrale Steuereinheit (12) umfasst, die sowohl mit der Messvorrichtung (8) verbunden ist, um Daten bezüglich der Glucosekonzentration im Blut zu erhalten, als auch mit einem Eingriffssystem (7, 11) zur Wiederherstellung der korrekten Glucosekonzentration im Blut des Patienten als Funktion der Daten von der Messvorrichtung (8); wobei das Eingriffssystem eine Dialyselösungs-Vorbereitungsvorrichtung (7) zur Dosierung der Glucose in die Dialyselösung und eine Insulininfusionsvorrichtung (11) zur Insulininjektion in den Patienten (P), der die Dialysebehandlung erhält, umfasst.

## Revendications

1. Machine de dialyse (1 ; 13) comprenant un filtre d'hémodialyse (2) ; une branche d'entrée (5) transportant une solution de dialyse vers ledit filtre (2) ; une branche de sortie (6) transportant la solution de dialyse depuis ledit filtre (2) ; une ligne d'artère (3) transportant le sang depuis un patient (P) jusqu'au filtre (2) ; une ligne de veine (4) transportant le sang du filtre (2) vers le patient (P) ; un certain nombre de pompes (3a, 9, 10, 16) pour faire circuler à la fois le sang et la solution de dialyse ; un dispositif d'ultrafiltration isolé (14) positionné le long de la ligne d'artère (3) du sang du patient, et à partir duquel part une ligne de ramification (15) où le plasma ultra-filtré ou l'eau de plasma circule ; et un dispositif de mesure de concentration de glucose (8) monté sur ladite ligne de ramification (15) ; ladite machine étant **caractérisée en ce qu'**elle comprend une unité de commande centrale (12) raccordée à la fois audit dispositif de mesure (8) pour recevoir des données concernant la concentration de glucose dans le sang, et à un système d'intervention (7, 11) pour rétablir la concentration de glucose correcte dans le sang du patient en fonction des données provenant du dispositif de mesure (8) ; ledit système d'intervention comprenant un dispositif de préparation de solution de dialyse (7) pour doser le glucose dans la solution de dialyse et un dispositif d'infusion d'insuline (11) pour injecter l'insuline dans le patient (P) qui est en dialyse.

2. Machine de dialyse (1 ; 13) comprenant un filtre d'hémodialyse (2) ; une branche d'entrée (5) transportant une solution de dialyse vers ledit filtre (2) ; une branche de sortie (6) transportant la solution de dialyse depuis ledit filtre (2) ; une ligne d'artère (3) transportant le sang depuis un patient (P) jusqu'au filtre (2) ; une ligne de veine (4) transportant le sang du filtre (2) au patient (P) ; un certain nombre de pompes (3a, 9, 10, 16) pour faire circuler à la fois le sang et la solution de dialyse ; et un dispositif de mesure de concentration de glucose (8 ; 8a ; 8b) monté sur ladite ligne de veine (3) ou ladite ligne d'artère (4) ou sur la ligne d'écoulement de dialysat ; ladite machine étant **caractérisée en ce qu'**elle comprend une unité de commande centrale (12) raccordée à la fois audit dispositif de mesure (8) pour recevoir des données concernant la concentration de glucose dans le sang, et à un système d'intervention (7, 11) pour rétablir la concentration du glucose correcte dans le sang du patient en fonction des données provenant du dispositif de mesure (8) ; ledit système d'intervention comprenant un dispositif de préparation de solution de dialyse (7) pour doser le glucose dans la solution de dialyse et un dispositif d'infusion d'insuline (11) pour injecter l'insuline dans le patient (P) qui est en dialyse.
